# EUROPEAN PATENT SPECIFICATION

(11) **EP 0 473 600 B1**
(45) Date of publication and mention of the grant of the patent: **13.07.1994**
(21) Application number: 90905699.6
(22) Date of filing: 21.03.1990
(51) Int. Cl.: A61B 5/117, A43D 1/02

(54) **PROCESS FOR ANALYSING FEET TO DETERMINE APPROPRIATE FOOTWEAR AND FOOT ANALYSIS APPARATUS THEREFOR**
VERFAHREN ZUM ANALYSIEREN VON FÜSSEN ZUR BESTIMMUNG GEEIGNETEN SCHUHWERKS UND DAFÜR GEEIGNETER FUSSANALYSEAPPARAT
PROCEDE D'ANALYSE DE LA FORME DES PIEDS PERMETTANT DE DETERMINER UNE CHAUSSURE APPROPRIEE, ET APPAREIL D'ANALYSE DU PIED PREVU A CET EFFET

(30) Priority: 21.03.1989 NL 8900693
(43) Date of publication of application: 11.03.1992
(73) Proprietor: SCHRIJVER, Floor, NL-3565 BE Utrecht (NL)
(72) Inventor: SCHRIJVER, Floor, NL-3565 BE Utrecht (NL)
(74) Representative: Van Assen, Jan Willem Bernard
(86) International application number: NL9000036
(87) International publication number: WO9011048

(56) References cited:
- DE-A- 3 711 539
- US-A- 1 543 747
- US-A- 2 998 983
- Medizinisch-Orthopädische Technik, vol.108, no.6, November/Dezember 1988 (Stuttgart DE) J.Grifka et al: "Die Trittspur als diagnostisches Hilfsmittel - Hinweise zu Durchführung und Auswertung", pages 228-231

## Description

The invention relates to a device for analysing the feet to determine appropriate footwear by taking foot imprints of the feet ,comprising a footform which possesses a print space provided with a adhesive layer ,adapted for applying at least one foot imprint, a cover foil to protect the adhesive layer, as well as an imprint foil , which foils are attached to one another along at least one side.

A device of this kind is described in US-A-2.998.983 This comprises superimposed sheets joined together along a single side.As a consequence hereof, this device known from US-A-2.998.983 requires tearing away of the imprint form and proper positioning over the foot form before use.In the device according to DE-A-3.711.539 the foil dedicated to the protection of the footform has to be torn away before an imprint is made, a second transparent protective foil for the protection of the imprint being permanently attached for subsequent protection.

The object of the invention is removing these problems and providing a foot analysis device with which a quick and effective imprint of the feet is possible and which allows an instantaneous comparison of the imprint with the 12 standardized types of foot giving an analysis of the foot in question.

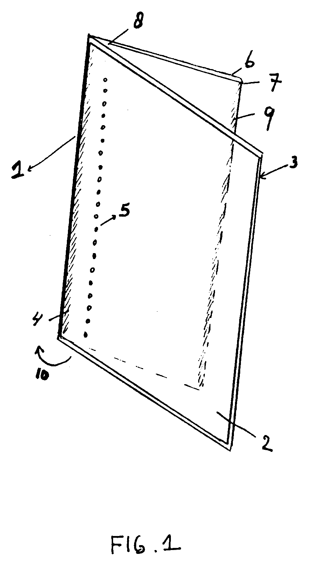

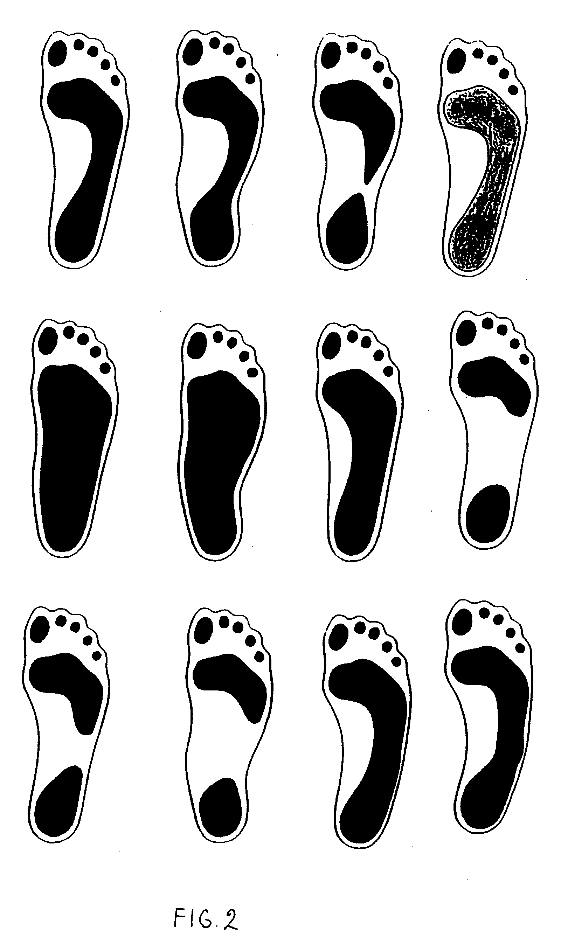

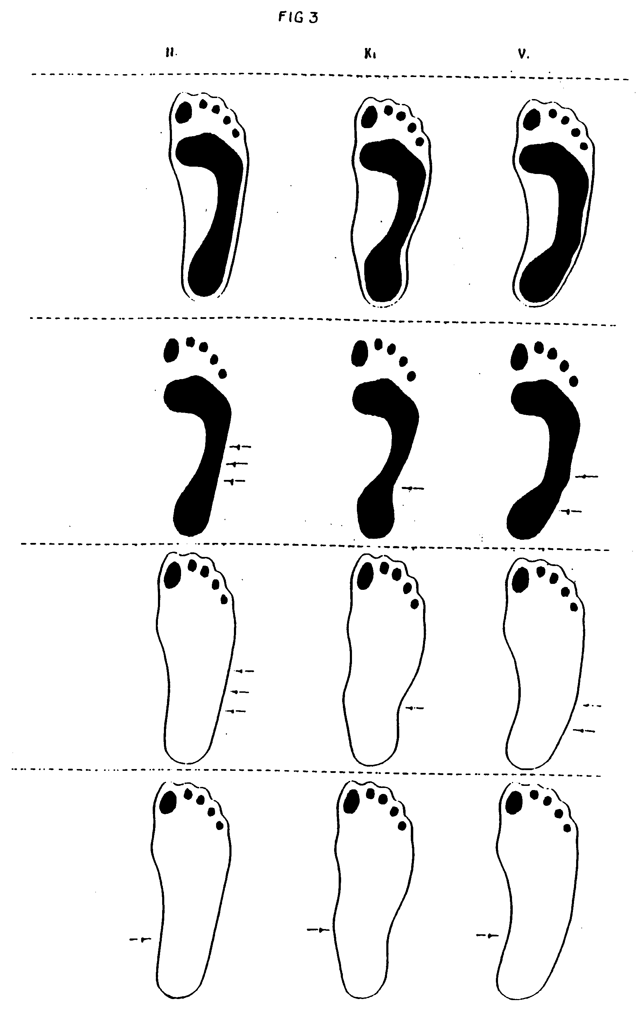

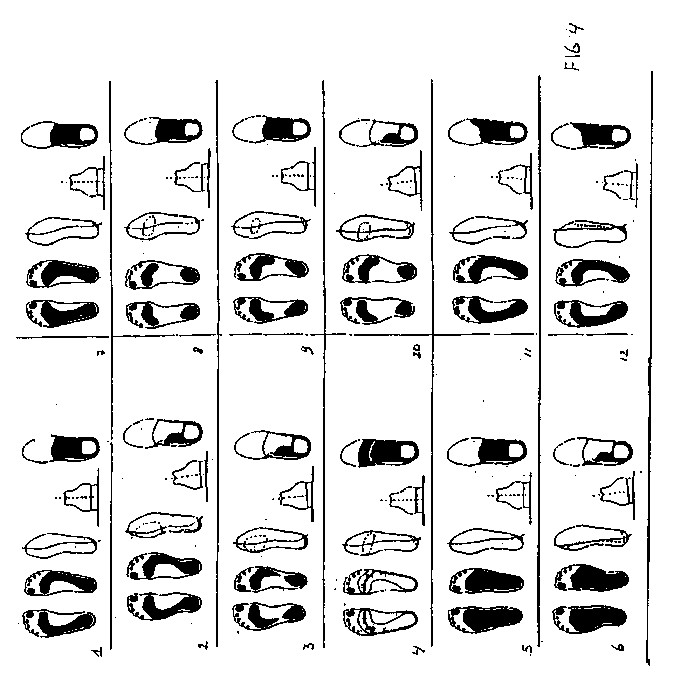

This object is reached according to the invention in that the protective foil is transparent,
the standardized types of footprints shown in Figure 2 are provided on the protective foil; and
the protective foil is attached to the longitudinal side of the device opposite to the side to which the "imprint foil" is connected.

These measures effectively simplify the complete operation because the transparent foil that was first used as a protection for the unused devive is after the imprint and removal of the printing layer or foil again a protection but now for the information foil with the imprint.

The podiatrist can then immediately analyse the foot imprints and chose the right footwear and/or the shoe inlays best suited for the person of which the foot imprints have been made.

The invention will now be further illustrated by the figures wherein
figure 1 shows a schematic presentation of a footform set,
figure 2 represents an illustration of the standard foot patterns,
figure 3 shows a general classification of the standard foot patterns and
figure 4 represents an illustration of either the footform or the protective foil with standard foot patterns and other information, especially the footline and the heel-position.

In figure 1 the footform set is indicated by 1. This comprises a cover foil 2 which can be provided with advertizing material and informations, to which is attached an imprint paper 3. The attachment (with glue) is indicated by the hatched line 4. The imprint paper contains a perforation line 5 parallel to the side and the attachment 4 by means of which the imprint paper after use can be torn off. The footform 6 is provided with a transparent protective foil 7 which generally has a smaller dimension than the form. The foil at the opposite side of 4 and 5 is attached to the form 9 and my be released by pulling away, starting from the side 8 of the form. On the form 6 an adhesive layer, not represented, is present, on the sides, besides the footimprint area, the standard foot models being arranged which serve for comparison. On the lower and upper side of the form possible further information and explanations can be taken up. The foot analysis set is so applied that in use the cover foil 2 and the imprint paper 3 are folded to the left according to figure 1 (see arrow 10), whereafter the transparent foil is pulled away from the footform starting at the non-fixed side (opposite 7). After the foil has been pulled away and the footform is free, the carbon layer is applied, which by the adhesive layer is uniformly adhering. Thereafter the footprints are taken by placing the foot on the carbon paper, which is arranged with the footform on a hard underground. Preferably the footform has such dimensions that two footprints, the right and left foot, can be taken beside each other. Thereafter the carbon paper is folded back again by pulling it cautiously off the footform with adhesive layer whereafter the transparent protective foil is again applied on the footform, now carrying the footprints. The carbon paper is no longer necessary and may be torn off along the perforation and removed. Thereafter one may make an analysis of the feet based on the standard patterns and the added text and give a suggestion for the right footwear and/or footinlay.

Figure 2 shows an arrangement of the foot patterns used, which are numbered 1 to 12 and which already are indicated as such in the description. The user will compare the footimprints made with the standard patterns which schematically represent substantially all possible types of foot and which were established based on a large amount of measurements with large numbers of feet. Therefore a reliable analysis is possible and because a direct relation is found between type of foot, way of walking and sensibility for injuries one can arrive at adapted improved footwear especially with adapted footinlays. It is to be understood that the set can be used separately or wound on a roll from which the user always can take away a copy.

As concerns figure 3 the following information is given. The types of foot as described can for a better understanding be underclassified in three categories. These are indicated neutral (N), kink (K) and varus (V). The particulars thereof are indicated in the figure with arrows in the three rows which are present below the foot imprint, i.e. the imprint pattern (indicated black), the outside of the circumferential line (lateral side) and the inside of the circumferential line (the medial side).

For neutral (N) holds: the outside of the heel part of the foot imprint to the forefoot is near enough straight or gradually curved to the inside (medial). The outside of the circumference to the forefoot is near enough straight or gradually curved to the inside (medial). The inside of the circumference just above the heel part is gradually curved outside (lateral).

For the kink position (K) holds: the outside of the heel part of the pressure pattern to the forefoot is more or less rotated to the inside (to the medial side) (kinked). The outside of the heel part of the circumference to the forefoot is more or less rotated inwards (to the medial side) (kinked). The inside of the circumference just above the heel part is more or less rotated to the inner side (to the medial side) (kinked).

For the varus position (V) holds that the outside of the heel part of the pressure pattern to the forefoot is rather extremely rotated to the outside (lateral side) (arched). The inside of the circumference is rather extremely rotated to the outside (lateral side) (arched). The three categories comprise the following foot models:
- N:: (1) normal, (2) hollow foot, (3) slightly-hollow foot, (4) highly-arched foot, (5) flat foot and (6) over-strained forefoot.
- K comprises:: (7) hollow kinkfoot, (8) kinkfoot, (9) slight kinkfoot, (10) flat kinkfoot.
- V comprises:: (11) slight varus position, (12) varus.

For the normal foot applies that the outside of the heel part to the forefoot is almost straight or slightly rotated inside. There is a good medial curve because of the space between foot and floor. For the hollow foot it is characteristic that the foot lengthwise is more hollow than one normally would expect. This can be seen by the lack of the imprint at the outside of the midfoot. As a consequence of this these feet are generally less flexible in their movements than normal feet. For the high-arched foot the total footimprint is more extensive than of the normal foot, especially at the outside (medial side). The foot is somewhat flatter in length. The flat foot is characterized by the lack of arches so that the strain is on the whole foot. For the over-strained forefoot there is an excessive pressure on one or more of the five metatarsal rays. This is made visual by a darker-coloured area. The kinked foot (K) comprises a hollow kinked foot which is a combination of a hollow foot and a kinked foot. The foot is over-rotated inside (kinked) and is also hollow. The kinked foot and the slight kinked foot have as characteristic that the outside of the heel part to the forefoot is no straight line but is too much rotated inside (kinked). The kink movement will increase when overloaded (over-pronation) having often disadvantages effects for the muscles, tendons etc. The flat kinked foot is a combination of the flat foot and the kinked foot. The foot is too much rotated (kinked) to the inside and the foot is flat. For both varus positions (V) applies that the load on the foot is greatest. The heel part is rather placed outwardly. During running problems arise because the foot is unrolling in a sideward rotation. It is evident that, based on the above characteristic specification, the possibility exists to design special inlay soles which can relieve foot complaints as much as possible.

In figure 4 an illustration is given of the standardized foot patterns with further information, which best is taken up on the cover foil. Apart of the already mentioned foot patterns beside each pair thereof from the left to the right in the drawing is indicated an impression of the bearing and gaitline, the position of the shoe seen from the back and the appropriate foot inlay. The foot impression being made by means of these details one can determine the type of foot and thus, by means of the drawing, select the appropriate foot inlay.

The wear and gaitline can be determined at the back part of the shoe and provide additional information on the feet to be studied. The gaitline is the average line which the feet make during walking. For the normal foot there is, as one sees from figure 4 under 1, first heel contact, thereafter the line is bending inside at the inner foot and next the foot is unrolled at the toe part. It can be seen at the kinked foot (figure 4,2) that this line is more strongly running inside at the heel. The foot as a whole is unrolling more through the inside; so each type has its own unrolling line (gaitline). The pieces coloured black and the dashed line in the sole pattern indicate where about the most wear of the sole is present.

The judgement of the wear and the deformation of the old shoe is to be considered as a check which relates to the foot imprint made. When one looks at the shoe from the back part, then for the normal foot the shoe will remain straight and as seen for the kinked foot the shoe will be deformed and be tilted inside. Thus one has at its disposable additional control possibilities which have to assure an optimal analysis. If for instance a foot imprint has been made of a varusfoot and the wearing and deformation of the shoe are those of a kinked foot, then one can be sure that the imprint was not made well.

With the form according to figure 4 apart from the analysis a correction is possible by means of the foot inlays drawn, which each belong to a normalized type of foot. These foot inlays are built up of different layers of flexible thermoplastic material (for instance EVA) which possess different hardneeses. The lower layer (the hardest layer) is indicated in black colour in figure 4. Especially the shape of this layer determines the correction. The other layers (mostly 2 or 3 layers), which are softer, are present on the harder lower layer and complete this in such a way that the foot inlay is conforming to the foot as concerns shape and circumference. In the white surface under the heel damping elements can be taken up. These foot inlays in practice can be provided with a suitable coat, colour etc. from which it appears to which type of foot they belong. After analysis one thus can already carry out a correction by selecting the appropriate foot inlay through the coding, for instance by taking this from a storage rack. It is to be understood that the mentioned normalized types of foot are developed independently of the dimensions of the foot. When selecting the foot inlay one will of course take account of the foot size and supply foot inlays in different dimensions.

Thus the invention provides an analysis system which is easily applicable and relatively cheap and which gives quickly practical results. It is to be understood that the choice of the different materials, like adhesive and protective foil, from the various known materials such as self-adhesive glues, dispersing glues and other types, as well as, related to the protective foil from different polymeric materials, lies within reach of the expert and that one can satisfy the condition that the carbon layer after use is removable without problems, and that equally the protective foil can be removed and again applied without problems. For this of course different folding methods are possible apart from that indicated in figure 1.

## Claims

1. Device for analyzing the feet to determine appropriate footwear by taking foot imprints of the feet ,comprising a footform (1) which possesses a print space provided with a adhesive layer ,adapted for applying at least one foot imprint, a protective foil (2) to protect the adhesive layer , as well as an imprint foil (3), which foils are attached to one another along at least one side , characterized in that
the protective foil (2) is transparent,
the standardized types of footprints shown in Figure 2 are provided on the protective foil (2); and
the protective foil (2) is attached to the longitudinal side of the device opposite to the side to which the imprint foil (3) is connected.

## Patentansprüche

1. Vorrichtung zum Analysieren von Füßen zur Bestimmung geeigneten Schuhwerks durch Abnahme von Fußabdrücken, umfassend eine Fußform (1), die ein mit einer adhäsiven Schicht, die Wenigstens einen Fußabdruck aufnehmen kann, einer Schutzfolie (2) zum Schutz dieser adhäsiven Schicht sowie einer Abdruckfolie (3) ausgestattetes Abdruckfeld aufweist, wobei die Folien entlang wenigstens einer Seite miteinander verbunden sind dadurch gekennzeichnet, daß die Schutzfolie (2) transparent ist, die standardisierten Typen von in Figur 2 dargestellten Fußabdrücken auf der Schutzfolie (2) vorgesehen sind und die Schutzfolie (2) an der Längsseite der Vorrichtung gegenüber der Seite, mit welcher die Abdruckfolie (3) verbunden ist, angebracht ist.

## Revendications

1. Dispositif d'étude du pied en vue de déterminer la chaussure adéquate par prise des empreintes du pied, comprenant une forme de pied (1) avec une surface imprimante composée d'une couche adhésive adaptée pour recevoir au moins une empreinte de pied, une feuille de protection (2) destinée à protéger cette couche adhésive, ainsi qu'une feuille imprimante (3), ces feuilles étant reliées ensemble le long au moins d'un côté, caractérisé en ce que la feuille de protection (2) est transparente, les empreintes type représentées à la figure 2 apparaissent sur la feuille de protection (2), et la feuille de protection (2) est attachée sur le côté longitudinal du dispositif à l'opposé du côté sur lequel est attachée la feuille imprimante (3).
